# EUROPEAN PATENT APPLICATION

(11) **EP 2 837 333 A2**
(43) Date of publication of application: **18.02.2015**
(21) Application number: 14171929.4
(22) Date of filing: 11.06.2014
(51) Int. Cl.: A61B 8/00, A61B 5/00

(54) **Biological optical measurement apparatus and ultrasound diagnostic apparatus**

(30) Priority: 12.06.2013 JP 2013123677; 26.12.2013 JP 2013270509
(71) Applicant: Kabushiki Kaisha Toshiba, Tokyo 105-8001 (JP)
(72) Inventor: Urano, Taeko, Tokyo, 105-8001 (JP); Takayama, Satoshi, Tokyo, 105-8001 (JP); Nakanishi, Tsutomu, Tokyo, 105-8001 (JP); Nakamura, Kenji, Tokyo, 105-8001 (JP)
(74) Representative: Hoffmann Eitle

(57) **Abstract**

According to one embodiment, a biological optical measurement apparatus includes a light emission unit, plural detectors, an image acquisition unit, and a calculation unit. The light emission unit is arranged on a surface of a subject and configured to substantially vertically emit light from an emission surface of the light emission unit to the surface of the subject. The plural detectors are arranged on a surface of a subject and configured to substantially vertically emit light from an emission surface of the light emission unit to the surface of the subject. The image acquisition unit acquires an image with respect to the subject.

## Description

### FIELD

Embodiments described herein relate generally to a biological optical measurement apparatus and an ultrasound diagnostic apparatus.

### BACKGROUND

There are various types of techniques of noninvasively measuring the inside of a body (subject). Optical measurement as one of these techniques has advantages in being free from the problem of exposure to radiation and being capable of selecting a compound as a measurement target by selecting a wavelength. A general biological optical measurement apparatus percutaneously emits light to the inside of a subject upon pressing a light emission unit against the skin surface of the subject, and measures the light which is transmitted through or reflected by the inside and emerges outside the subject upon being transmitted through the skin again, thereby calculating various types of biological information based on the measurement. The ground for determining the presence of an abnormal tissue in a subject by optical measurement is the difference in absorption coefficient between the abnormal tissue and a normal tissue. That is, since the absorption coefficient of the abnormal tissue in the subject differs from that of the normal tissue, the amounts of light detected differ in accordance with the difference in the amount of light absorbed. That is, solving this as an inverse problem from the amount of detected light can obtain the absorption coefficient of the abnormal tissue. The characteristics of the abnormal tissue can be discriminated from the obtained absorption coefficient. In addition, a measurement position and a depth are analyzed from measured light. This analysis technique includes a technique (space-resolved method) of adjusting the distance between a light emission unit (to be simply referred to as a light source hereinafter) and a detector, a technique (time-resolved method) of obtaining depth information from the difference in arrival time of light by using a light source whose intensity changes with time, and a method combining these techniques. These analysis methods implement a biological optical measurement apparatus capable of acquiring high-quality signals. However, visualization of information based on light in a subject has a problem in low spatial resolution. In addition, in order to obtain correct position information from the detection result of reflected light, it is necessary to compute many data by using a complex algorithm. That is, it is not possible to perform real-time determination.

Highly feasible applications of biological optical measurement include breast cancer examination (see Jpn. Pat. Appln. KOKAI Publication No. 2005-331292). As described above, however, since optical measurement itself has problems in resolution and analysis time, it is preferable to use a scheme of improving examination performance by combination with other modalities. Under the circumstances, the present inventors have proposed a scheme of compensating for the low spatial resolution of light by using the morphological information obtained from ultrasound echo apparatus (see Jpn. Pat. Appln. KOKAI Publication No. 2007-020735). It is expected to be able to discriminate morphological characteristics in a tissue and the component distribution of a morphological characteristic portion within a shorter time than the conventional scheme by using this scheme. However, there is still room for improvement in real-time determination.

Breast cancer is one of the main causes of mortality among women. Breast cancer screening and early diagnosis are highly valued in terms of reducing a mortality rate and suppressing a healthcare cost. A current method includes palpation of breast and X-ray imaging (mammography) for searching for suspicious tissue deformation. If there is a suspicious region in a mammogram, ultrasound imaging is performed, and surgical tissue biopsy is further performed. A series of these examinations requires much time to reach a final conclusion. In addition, there is a problem among premenopausal young women in that a density of mammary gland is high and it is difficult to obtain satisfactory sensitivity in X-ray imaging. Therefore, the significance of screening by ultrasound imaging is large especially among young women.

In general, in ultrasound imaging, a qualified operator collects ultrasound still images, and a specialized radiogram interpreter (plural radiogram interpreters in some case) performs determination from morphological information on images. In medical examination, the number of subjects to be screened by one operator is limited to a maximum of 50 per day in consideration of the risk of oversight due to the fatigue and loss of concentration of the operator.

In capturing ultrasound images, when collecting still images depicting morphological characteristics, the knowledge and experiences of the operator are very important. In order to perform accurate and quick screening, the operator needs to be skilled. For example, the standard examination time per subject is 5 min to 10 min, but some operators may require more times depending on their skills. That is, in screening using a current ultrasound imaging technique, the accuracy of image collection may vary depending on the levels of skill of operators. Furthermore, when collecting images, the operator is required to always pay attention to images and perform determination independently. For this reason, a heavy mental pressure is imposed on even a skilled operator. A scheme of collecting all image information from a moving image is available. In this case, however, a heavy pressure is imposed on a radiogram interpreter.

In order to solve problems in such ultrasound image diagnosis, a scheme of reducing the pressure on a technician is proposed by guiding an ultrasound probe to the measurement position in a planar direction based on the metabolic information of a subject which is obtained by biological optical measurement (see Jpn. Pat. Appln. KOKAI Publication No. 2009-077931). In this scheme relatively easy detection and discrimination of an abnormal region within a short time in ultrasound imaging is possible as compared with a related art.

There is still room, however, for improvements in a conventional biological optical measurement method, a biological optical measurement apparatus using the method, and ultrasound probe position guiding in ultrasound imaging.

### BRIEF DESCRIPTION OF THE DRAWINGS

FIG. 1 is a block diagram showing the arrangement of a biological optical measurement apparatus 4 including an optical probe 40 and an optical measurement processing unit 42;
FIG. 2 is a block diagram showing the arrangement of an ultrasound diagnostic apparatus 1 incorporating the biological optical measurement apparatus 4 according to this embodiment;
FIG. 3 is a side view showing an example of a light guide portion 402 using a tapered structure;
FIG. 4 is a cross-sectional view showing a situation in which the light guide portion 402 shown in FIG. 4 is arranged on the surface of the subject;
FIG. 5 is a view showing an example of the layout of a light emission unit 400, plural detectors 401, and a light guide portion 402 on a subject surface;
FIG. 6 is a view for explaining the positional relationship between the light emission unit 400 and the detector 401 when using the inclined structure of a detector which corresponds to the light detection distance;
FIG. 7 is a view showing an example of a cross-section of the optical probe 40 with the inclined structure of the detector which corresponds to the light detection distance;
FIG. 8 is a sectional view of the optical probe 40 shown in FIG. 7 taken along A - A;
FIG. 9 is a view for explaining a simulation using the optical probe 40;
FIGS. 10, 11, and 12 are views each showing the distribution of light intensities (relative intensities to the values measured in the vertical direction when inclination angle θ = 0), for each inclination angle θ and each rotational angle φ, which are obtained by a simulation;
FIGS. 13A and 13B are graphs showing X-axis direction components and Y-axis direction components of the light intensities obtained by measurement using the optical probe 40 (FIGS. 7 and 8);
FIG. 14 is a view showing the light guide portion 402 viewed from the subject contact surface side of the probe P;
FIG. 15 is a graph showing the intensities of detected light with and without the light guide portion 402;
FIG. 16 is a view showing an example of the probe P in which the opening angle φ of each light guide portion 402 and the inclination angle θ of each detector 401 are adjusted in accordance with a light detection distance R;
FIGS. 17A and 17B are graphs each associated with the opening angle φ obtained by measurement using the optical probe 40 (FIGS. 7 and 8); and
FIG. 18 is a flowchart showing guidance processing for an ultrasound probe using the biological optical measurement apparatus 4.

### DETAILED DESCRIPTION

In general, according to one embodiment, a biological optical measurement apparatus includes a light emission unit, plural detectors, an image acquisition unit, and a calculation unit. The light emission unit is arranged on a surface of a subject and configured to substantially vertically emit light from an emission surface of the light emission unit to the surface of the subject. The plural detectors are arranged on a surface of a subject and configured to substantially vertically emit light from an emission surface of the light emission unit to the surface of the subject. The image acquisition unit acquires an image with respect to the subject. The calculation unit calculates a relative position between a position of the subject corresponding to the acquired image and a position of the detection of the intensities of light by the plural detectors. To make a description more specific, a biological optical measurement apparatus used in ultrasound image diagnosis will be described. However, without being limited to this example, the biological optical measurement apparatus according to this embodiment can also be used as an independent apparatus. In addition, in the embodiment, as will be described later, a biological optical measurement apparatus 4 incorporated in an ultrasound diagnostic apparatus 1 (constituting an integrated structure with the ultrasound diagnostic apparatus 1) will be explained. However, without being limited to this example, the biological optical measurement apparatus and the ultrasound diagnostic apparatus may be used as separate structures.

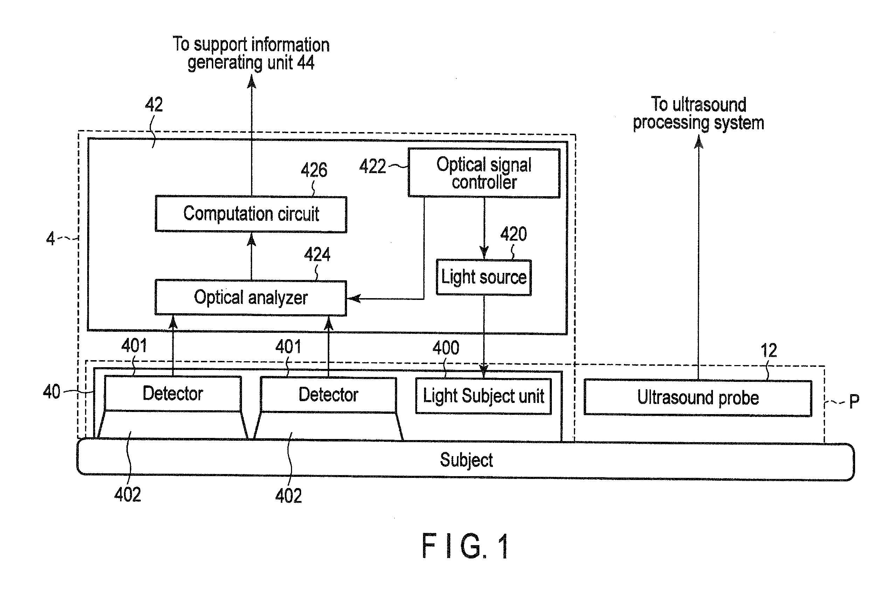

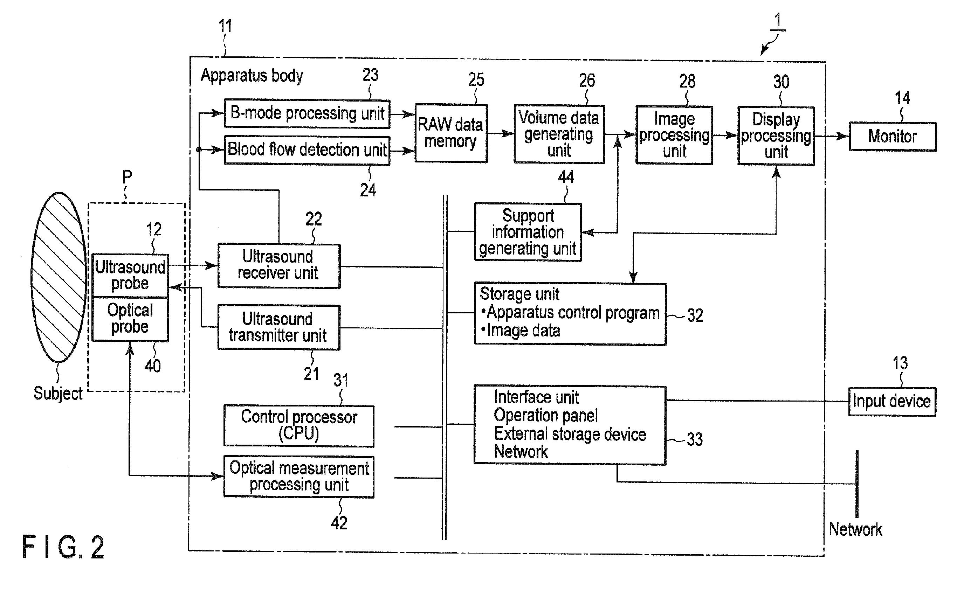

FIG. 1 is a block diagram showing the arrangement of the biological optical measurement apparatus 4 including an optical probe 40 and an optical measurement processing unit 42. FIG. 2 is a block diagram showing the arrangement of the ultrasound diagnostic apparatus 1 incorporating the biological optical measurement apparatus 4 according to this embodiment. Note that in the following description, the same reference numbers denote constituent elements having almost the same functions and arrangements, and a repetitive description will be made only when required.

The biological optical measurement apparatus 4 includes the optical probe 40, the optical measurement processing unit 42, and a support information generating unit 44 which generates support information for supporting the guidance operation of an ultrasound probe 12.

The optical probe 40 includes at least one light emission unit 400, plural detectors 401, and a light guide portion 402 for improving photodetection efficiency. The light emission unit 400 almost vertically emits light (near-infrared light) generated by a light source 420 to a subject. The detector 401 includes plural detection elements, each of which has a contact surface formed from, for example, an end portion of an optical fiber and photoelectrically converts reflected light from the inside of a subject which is input from the contact surface through the light guide portion. It is possible to use, as a detection element, for example, a light-detecting element such as a photodiode or phototransistor, CCD, APD, or photomultiplier tube. An optical matching layer may be provided on the surface of the light emission unit 400 which comes into contact with a subject.

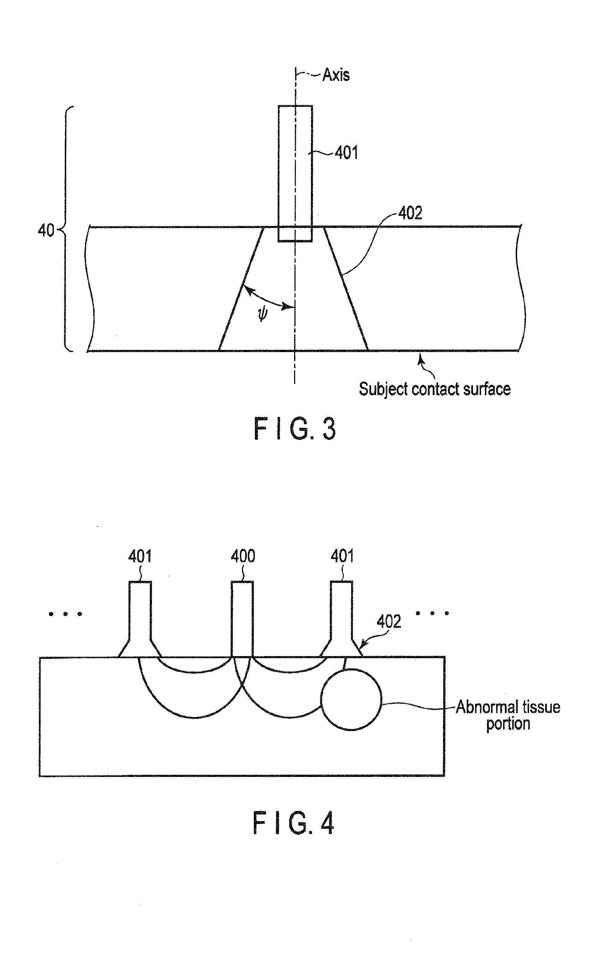

As shown in FIG. 3, the light guide portion 402 is provided between each detector 401 and a subject surface, and has a conical structure (for example, a tapered structure) whose opening area on the subject surface side is larger than that on the detection surface side of the detector 401. The arrangement of the light guide portion 402 will be described later in detail.

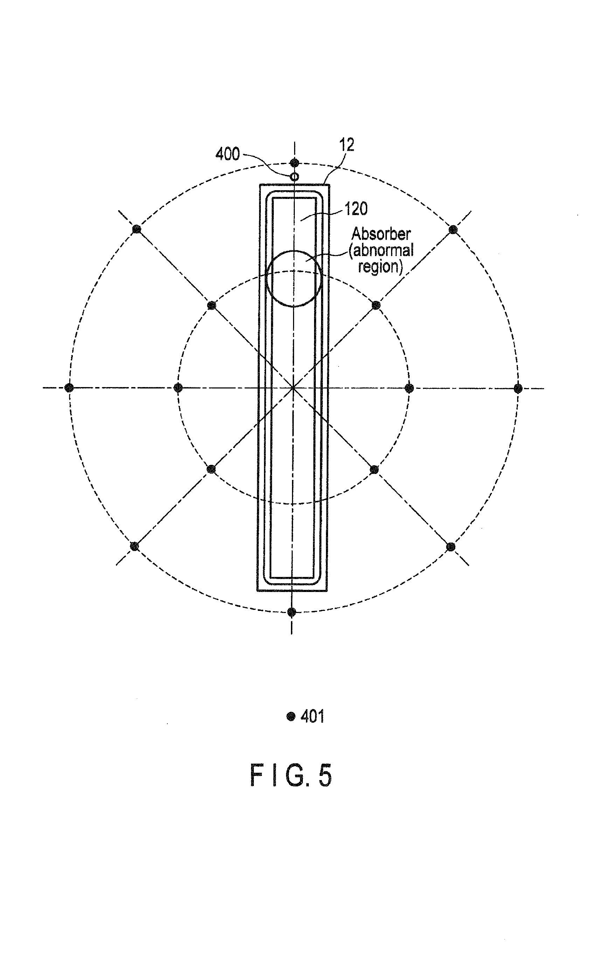

FIG. 4 is a cross-sectional view showing a situation in which the light guide portion 402 shown in FIG. 3 is arranged on the surface of the subject. FIG. 5 is a view showing an example of the layout of the light emission unit 400, the plural detectors 401, and the light guide portion 402 on a subject surface. FIG. 5 is a view showing a probe P viewed from the subject contact surface side. Referring to FIGs. 4 and 5, an abnormal tissue portion is, for example, shown as a spherical absorber. However, when applying this embodiment, an abnormal tissue portion is not limited to this example. Light applied from the light emission unit 400 is repeatedly reflected (scattered) inside the subject and emerges again outside the subject. Since the light emerging outside the subject in this manner has been repeatedly scattered inside the subject in this manner, the intensity of the light is greatly attenuated as compared with the incident light, and the direction of the light is also dispersed. For this reason, for example, the intensity of light emerging from a given point is greatly attenuated as compared with the incident light.

In order to evaluate the presence/absence and characteristics of an abnormal tissue portion in biological optical measurement, it is necessary to compare the intensity of emerging light in the presence of an abnormal tissue portion with that in the absence of an abnormal tissue portion. In addition, when biological optical measurement is to be used in ultrasound image diagnosis, in order to detect a proximity state with respect to a position at which ultrasound image should be measured, show the operator the proximity state, and guide the ultrasound probe (and the optical probe 40) to the position, the light intensities detected in the scanning of the optical probe 40 need to have S/N ratios high enough to perform computation. As will be described later, the biological optical measurement apparatus according to this embodiment can dramatically increase S/N ratio as compared with the related art by using the light guide portion 402.

The optical measurement processing unit 42 includes the light source 420, an optical signal controller 422, an optical analyzer 424, and a computation circuit 426. The light source 420 is a light-emitting element such as a semiconductor laser, light-emitting diode, solid-state laser, or gas laser, which generates light having a wavelength that exhibits a low biological absorption (for example, light in the wavelength range of 600 nm to 1,000 nm, which is a called a biological window) and light having a specific wavelength that exhibits an increase in absorption amount in an abnormal region (for example, light in the wavelength range of 750 nm to 850 nm, which falls within the wavelength range called the biological window, with hemoglobin in blood absorbing light in the range). Light generated by the light source 420 is supplied to the light emission unit 400 through a light guide portion (or directly through a space) formed from an optical fiber or a thin-film light waveguide. Note that the light source 420 may be united with the light emission unit 400.

The optical signal controller 422 dynamically or statically controls the biological optical measurement apparatus 4. The optical signal controller 422 controls the light source 420 to apply light from the light emission unit 400 at a predetermined timing, frequency, intensity, and intensity variation period T under the control of a control processor 31 of the ultrasound diagnostic apparatus 1. In addition, the optical signal controller 422 controls the optical analyzer 424 so as to execute optical analysis processing at a predetermined timing.

The optical analyzer 424 amplifies the analog signal input from the detectors 401 and then converts the signal into a digital signal. In addition, the optical analyzer 424 analyzes a change in the intensity of detected light among the detectors 401.

The computation circuit 426 calculates the degree of contact between the ultrasound probe 12 and a subject surface, the depth of an abnormal region exhibiting a predetermined light absorption coefficient in the subject (for example, a region which absorbs light with a specific wavelength more than a normal tissue) from the subject surface, and the three-dimensional position and distance of the abnormal region with reference to a predetermined position (for example, the center of the light emission unit 400 or an ultrasound transmitter/receiver surface 120) based on a change in the intensity of detected light between the detectors 401. The calculation result obtained by the computation circuit 426 is sent to the support information generating unit 44.

The support information generating unit 44 generates support information for instructing and guiding the position, posture, direction, and the like of the ultrasound probe 12 based on the determination result and calculation result obtained by the computation circuit 426 in order to bring the transmitter/receiver surface of the ultrasound probe 12 into intimate contact with the subject. The generated support information is displayed in a predetermined form.

The ultrasound diagnostic apparatus 1 will be described next. As shown in FIG. 2, the ultrasound diagnostic apparatus 1 includes the ultrasound probe 12, an input device 13, a monitor 14, an ultrasound transmitter unit 21, an ultrasound receiver unit 22, a B-mode processing unit 23, a blood flow detection unit 24, a RAW data memory 25, a volume data generating unit 26, an image processing unit 28, a display processing unit 30, a control processor unit (CPU) 31, a storage unit 32, and an interface unit 33.

The ultrasound probe 12 and the optical probe 40 constitute a probe P. The ultrasound probe 12 is a device (probe) which transmits ultrasound waves to a subject, typically a subject and receives reflected waves from the subject based on the transmitted ultrasound waves. The ultrasound probe 12 includes, on its distal end, an array of plural piezoelectric transducers, a matching layer, a backing member, and the like. For example, as shown in FIG. 5, the light emission unit 400 and detectors 401 of the biological optical measurement apparatus 4 are arrayed in a predetermined form near the ultrasound transmitter/receiver surface of the ultrasound probe 12. The input device 13 is connected to an apparatus body 11 and includes various types of switches, buttons, a trackball, a mouse, and a keyboard which are used to input various types of instructions for operating the ultrasound diagnostic apparatus 1 and the biological optical measurement apparatus. The monitor 14 displays morphological information and blood flow information in the subject and the support information generated by the support information generating unit 44 in a predetermined form.

The ultrasound transmitter unit 21 generates trigger pulses for the formation of transmission ultrasound waves at a predetermined rate frequency fr Hz (period: 1/fr sec), gives a predetermined delay time, and then applies a driving pulse to the probe 12. The ultrasound receiver unit 22 amplifies an echo signal received via the probe 12 for each channel, performs A/D-conversion of each signal, and then performs addition processing for the signals upon giving necessary delay times to the respective signals. The B-mode processing unit 23 receives an echo signal from the receiver unit 22, and performs logarithmic amplification, envelope detection processing, and the like for the signal to generate data whose signal intensity is expressed by a luminance level. The blood flow detection unit 24 extracts a blood flow signal from the echo signal received from the receiver unit 22, and generates blood flow data (blood flow information such as an average velocity, variance, or power).

The RAW data memory 25 generates RAW data by using plural B-mode data received from the B-mode processing unit 23 and the blood flow detection unit 24. The volume data generating unit 26 generates B-mode volume data and blood flow volume data by executing RAW-voxel conversion including interpolation processing in consideration of spatial position information. The image processing unit 28 performs predetermined image processing such as volume rendering, MPR (Multi Planar Reconstruction), and MIP (Maximum Intensity Projection) for the volume data received from the volume data generating unit 26. The display processing unit 30 executes various types of processes associated with a dynamic range, luminance (brightness), contrast, γ curve correction, RGB conversion, and the like for various types of image data generated/processed by the image processing unit 28. The control processor unit 31 has the function of an information processing apparatus (computer) and controls the operation of each constituent element. The storage unit 32 stores a dedicated program, captured volume data, diagnosis information (patient ID, findings by doctors, and the like), a diagnostic protocol, transmitter/receiver conditions, captured images, and other data groups.

The interface unit 33 is an interface associated with the input device 13, a network, and a new external storage device (not shown). An external biological optical measurement apparatus can also be connected to the ultrasound diagnostic apparatus main body 11 via the interface unit 33. The interface unit 33 can transfer, via a network, data such as ultrasound images, analysis results, and the like obtained by this apparatus to another apparatus.

### (Inclined Structure of Detector Which Corresponds to Light Detection distance)

The inclined structure of the detector which corresponds to a light detection distance of the biological optical measurement apparatus 4 will be described next. This structure is designed to set individually, for each detector 401, the angle (inclination angle θ) defined by the central axis of the light emission unit 400 (the optical axis of light applied from the light emission unit 400) and the central axis of each detector 401 in accordance with the light detection distance R (the distance from the light incidence position of light applied from the light emission unit 400 to each detector 401).

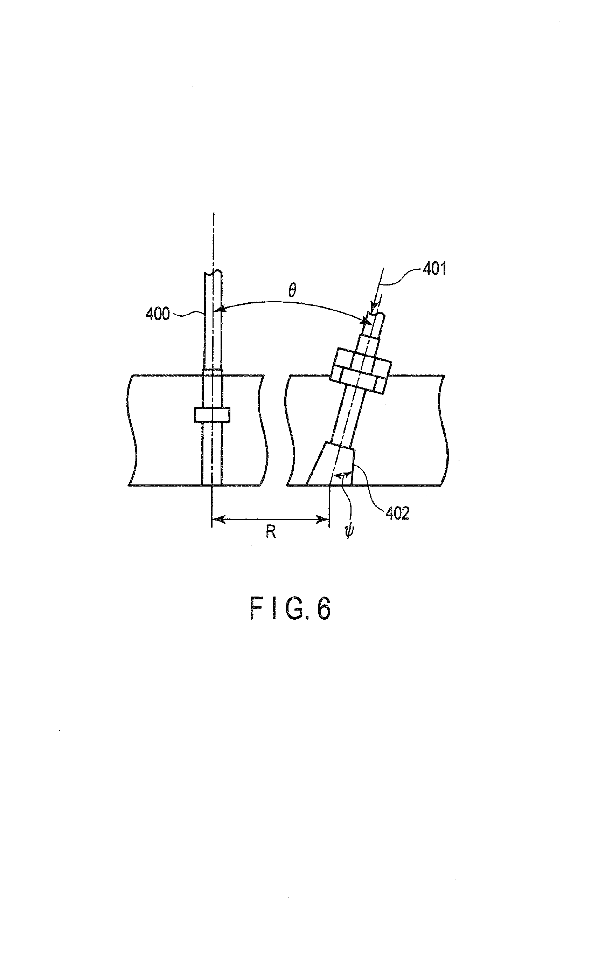

FIG. 6 is a view for explaining the positional relationship between the light emission unit 400 and the detector 401 when using the inclined structure of the detector which corresponds to the light detection distance. As shown in FIG. 6, each detector 401 is provided such that the central axis of the detector 401 is inclined by the inclination angle θ with respect to the central axis of the light emission unit 400 in accordance with the light detection distance R from the light emission unit 400. In this case, the inclination angle θ takes a value equal to or more than 0° and less than 90°. If, for example, inclination angle θ = 0°, the central axis of the detector 401 is set to be substantially parallel to the central axis of the light emission unit 400. Although it depends on the wavelength of light to be used for measurement, when light having a wavelength band (for example, 765 nm) called a biological window is used as incident light, the inclination angle θ is individually provided in accordance with the light detection distance R such that when the light detection distance R is within 5 mm, the inclination angle of the detector 401 is given as θ = 0°, whereas when the light detection distance R is larger than 5 mm, the inclination angle θ of the detector 401 is set to, for example, a value larger than 0° and less than 90°.

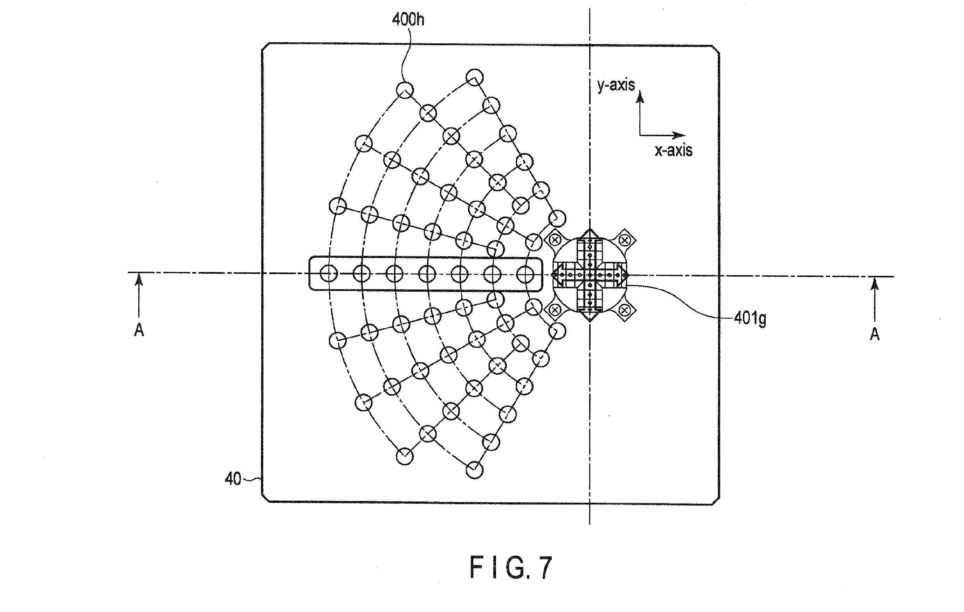

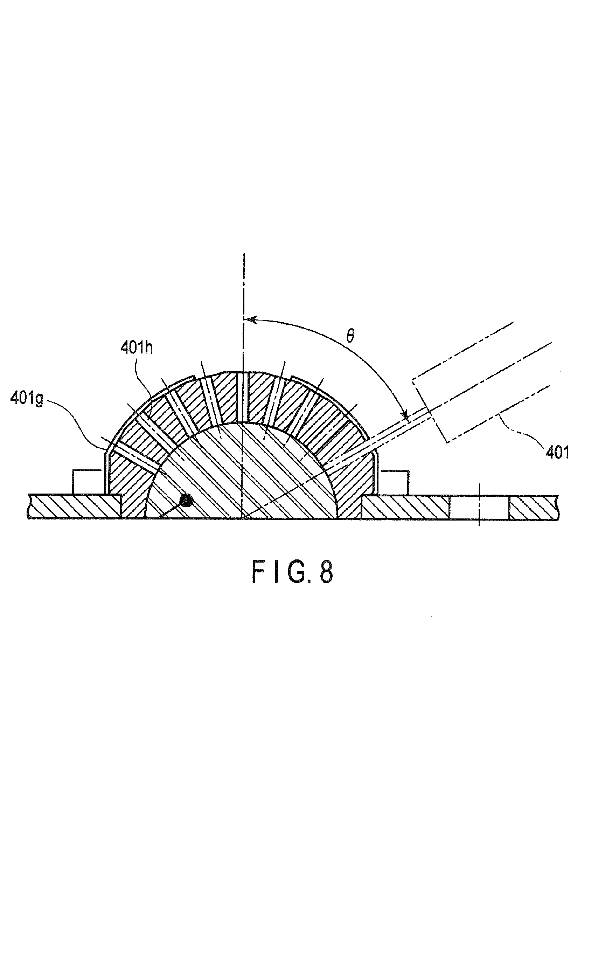

FIG. 7 is a view showing an example of a cross-section of the optical probe 40, manufactured experimentally by the present inventors, in which a detector has an inclined structure corresponding to the light detection distance. FIG. 8 is a sectional view of the optical probe 40 shown FIG. 7 taken along A - A. As shown in FIGS. 7 and 8, the optical probe 40 includes plural guide holes 400h and a detector guide block 401g. As shown in FIG. 7, the plural guide holes 400h are arranged at predetermined intervals on the circumferences of concentric circles centered on the detector guide block 401g. As shown in FIG. 8, the detector guide block 401g has a hemispherical (dome-like) housing provided with plural detector guide holes 401h whose inclination angles θ differ in increments of, for example, 15°. The optical probe 40 makes it possible to adjust the distance (i.e., the light detection distance R) between the light emission unit 400 and the detector guide block 401g by changing the guide hole 400h in which the incident fiber of the light emission unit 400 is inserted. In addition, changing the detector guide hole 401h in which the light-detecting fiber of the detector 401 is inserted can adjust the angle (i.e., the inclination angle θ) defined by the central axis of the light emission unit 400 and the central axis of the detector 401.

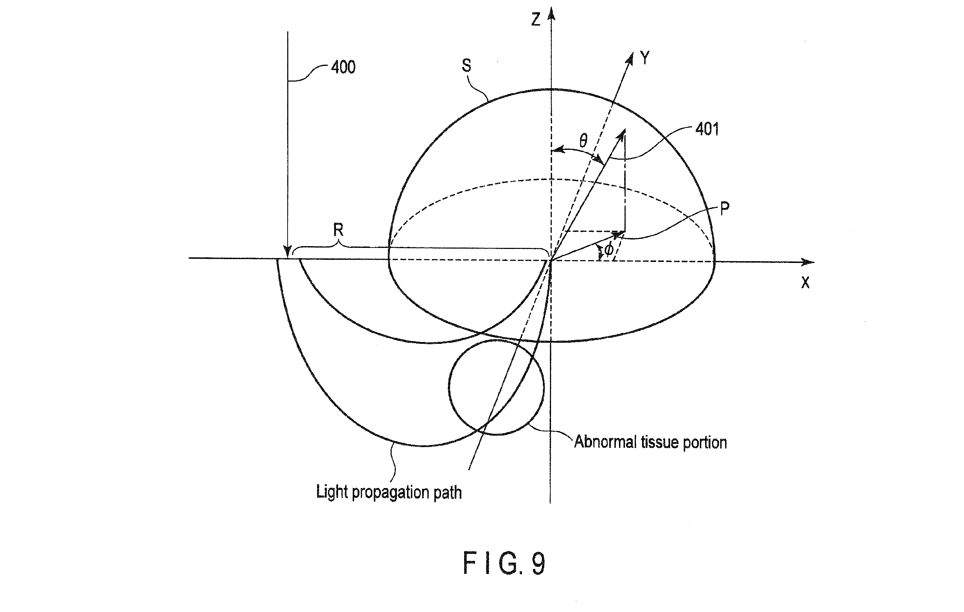

The present inventors performed the following a Monte Carlo simulation by using the optical probe 40 to obtain the optimal inclination angle θ corresponding to the light detection distance R. As shown in FIG. 9, the experiment corresponding to the simulation is as follows; the light detection distance R was set as a fixed value, and the inclination angle θ was changed from -60° to +60° in increments of 15° by changing the detector guide hole 401h in which the light-detecting fiber was inserted. In addition, the present inventors changed a rotational angle φ defined by an extended line of a straight line (the X-axis in FIG. 9) connecting the center of the detector 401 to the center of the light emission unit 400 and a straight line P obtained by projecting the central axis of the detector 401 on a subject surface from -60° to +60° in increments of 15°, and measured the light intensity detected by the detector 401. In this simulation, the inclination angle θ was changed from -90° to +90°. Note that incident light (765 nm) was made to vertically emit a sample surface, and a reflected light intensity was measured at a position 10 mm apart from an incident position. The sample was obtained by dispersing a scattering agent and a pigment in a resin and solidifying the resultant structure so as to match its scattering coefficient and absorption coefficient with those of the subject.

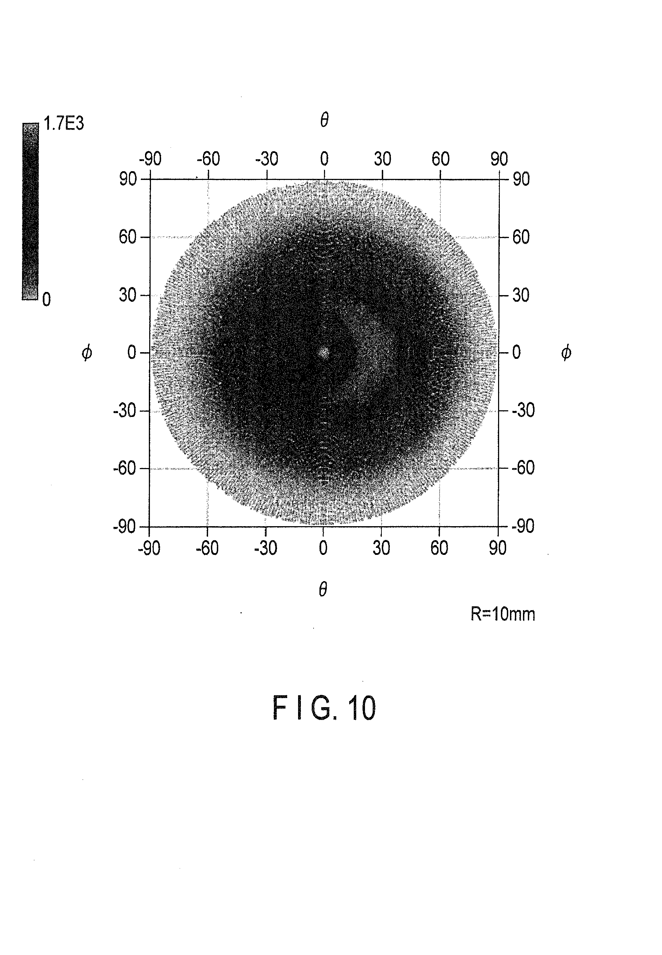

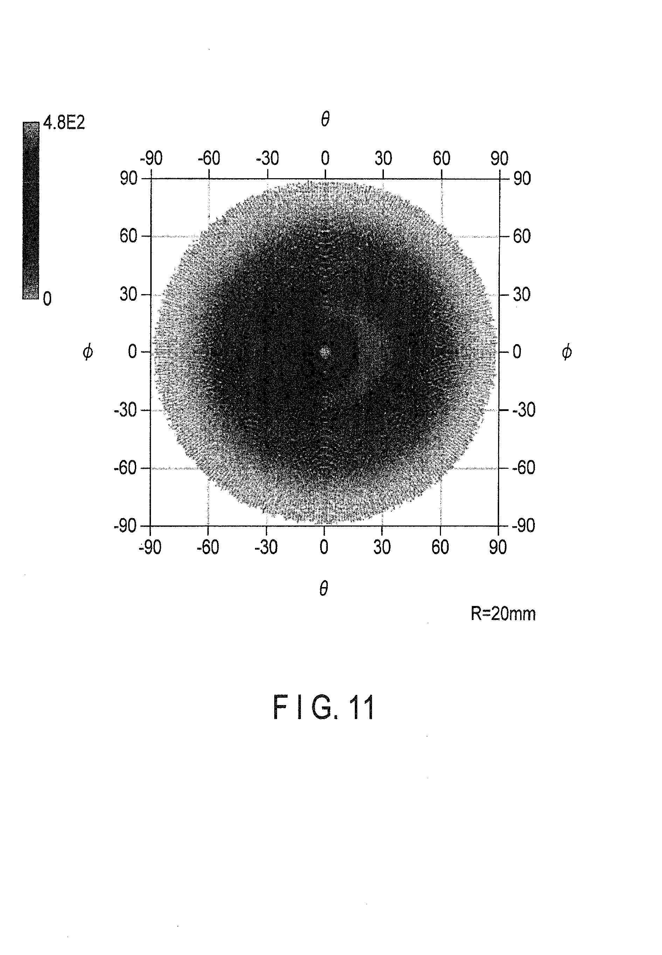

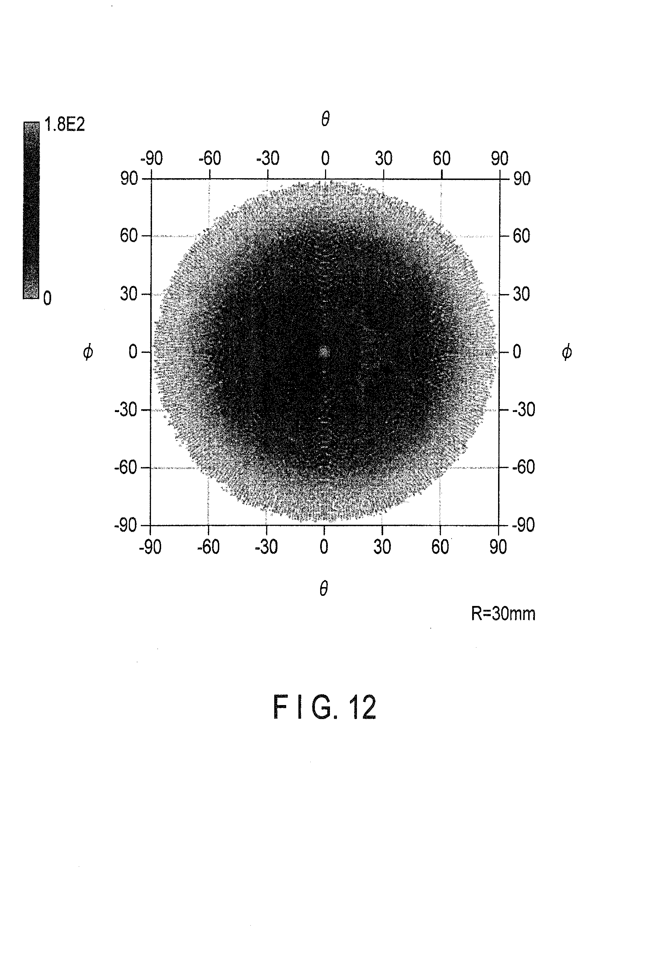

FIGS. 10, 11, and 12 show the distributions of light intensities (intensities relative to the values measured in the vertical direction with inclination angle θ = 0) obtained by the above simulation for each inclination angle θ and each rotational angle φ when the light detection distances are respectively set as R = 10 mm, 20 mm, and 30 mm. As is obvious from the comparison between FIGS. 10, 11, and 12, as the light detection distance R increases, the dependence on the inclination angle θ decreases (that is, the ratio of obliquely emerging light decreases), and the average emerge angle approaches 0°. In addition, as is obvious from the simulation result shown in FIG. 12, the angle dependence of emerge light remains at least up to light detection distance R = 30 mm, and the detection efficiency is improved by inclining the detector 401.

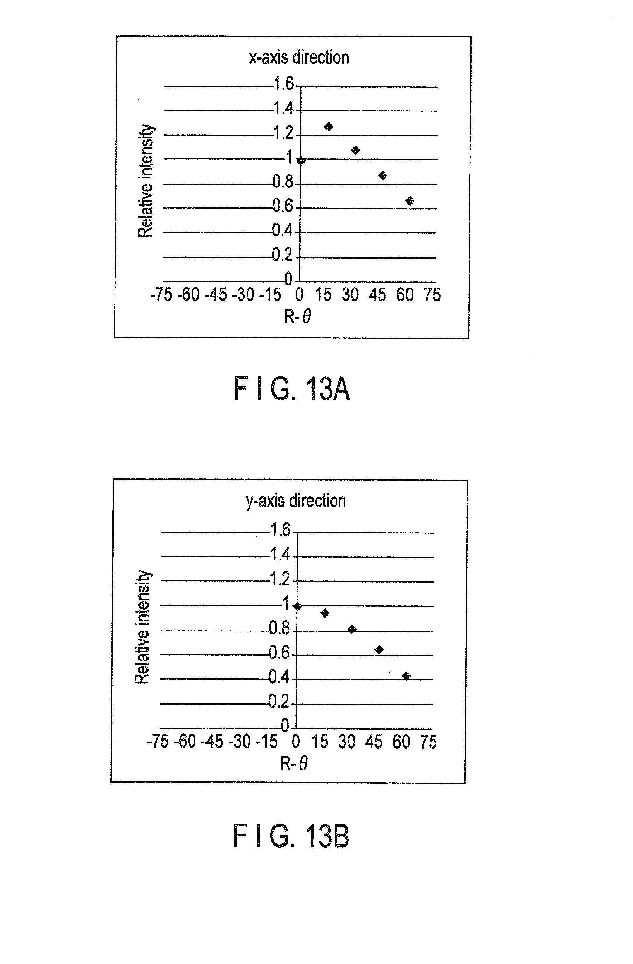

FIGs. 13A and 13B are graphs showing X-axis direction components and Y-axis direction components of the light intensities obtained by the experiments shown in FIGS. 7 and 8. FIGS. 13A and 13B indicate that the light detected when inclination angle θ = 15° has the maximum intensity, that is, a significantly high intensity, with respect to the intensity of light detected when inclination angle θ = 0. The experimental result shown in FIGS. 13A and 13B is a rough data in increments of 15°. In order to determine properly an angle range in which a signal can be increased as compared with that obtained when the inclination angle is 0°, the simulation result can be utilized as shown in FIGs. 10, 11, and 12. From FIGs. 10, 11, and 12, the range of the inclination angles θ in which light has a sufficiently high intensity compared with the case at the inclination angle θ = 0 is, for example, from 10° to 40°. Especially, the effect of increasing light intensity is large in the range from 15° to 35°. This result is consistent with the result obtained from the experiment as shown in FIGS. 13A and 13B.

### (Light Guide Function Using Tapered Structure)

A light guide function using the tapered structure of the biological optical measurement apparatus 4 will be described next. As shown in FIG. 6, this function is implemented by providing, between at least one detector 401 and a subject surface, the light guide portion 402 having a structure (e.g., a tapered structure) in which the opening area on the subject surface side is larger than that on the detection surface side of the detector 401. Note that an optical fiber may be provided between the detection surface of the detector 401 and the light guide portion 402. In this case, the opening area on the light guide portion side of the optical fiber is smaller than that on the subject surface side of the light guide portion. Providing the light guide portion 402 having such a structure on the detection surface side of the detector 401 can efficiently guide (or focus) not only light parallel to the axis of the detector 401 but also light which is repeatedly scattered inside the subject and is not parallel to the axis of the detector 401 to the detection surface of the detector 401. As a consequence, the light detection efficiency of each detector 401 dramatically improves.

Note that the opening area of the light guide portion 402 depends on an opening angle φ defined as shown in FIG. 6 (or FIG. 3). The opening angle φ of each light guide portion 402 may differ in accordance with the light detection distance R.

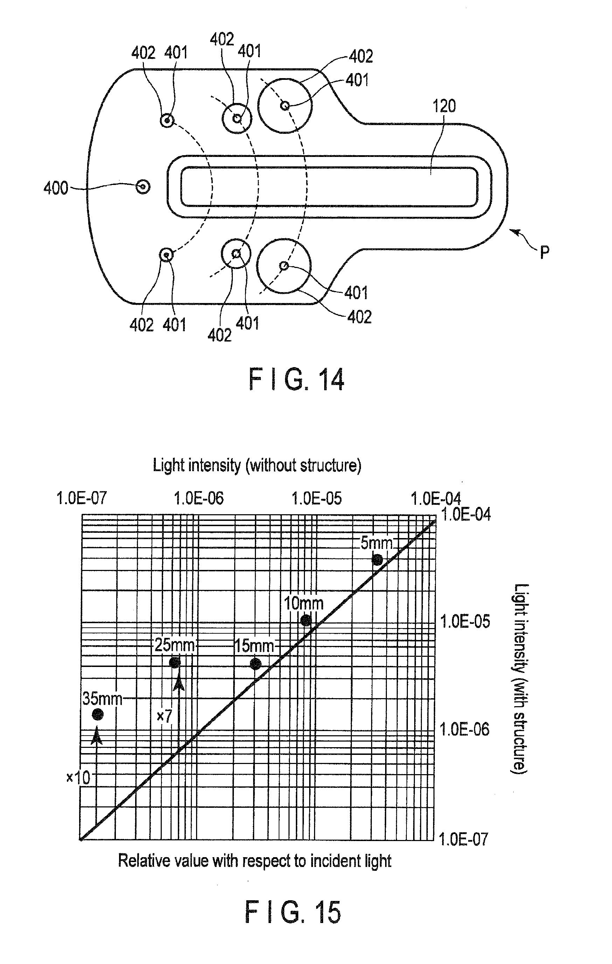

FIG. 14 is a view of the probe P viewed from the subject contact surface side, and shows how the opening of the subject contact surface of the light guide portion 402 increases with an increase in the light detection distance R. Referring to FIG. 14, each dotted line indicates the distance from the light emission unit 400 to each detector 401. This arrangement can dramatically improve the light guide efficiency at a position apart from a light emission position.

FIG. 15 is a graph showing the intensities of light detected when the light guide portion 402 is mounted compared with that when the light guide portion 402 is not mounted. The present inventors verified and confirmed that, as shown in FIG. 15, the light guide portion 402 increased light intensity by a factor of 1.2 to 1.3 when the light detection distance is 5 mm to 15 mm, whereas it increased light intensity by a factor of 6.8 when the light detection distance is 25 mm, and by a factor of 10 when the light detection distance is 35 mm. In addition, such an effect was recognized when the diameter of the opening area of the subject contact surface of the light guide portion 402 was about 5 mmφ, which allows fabrication for a minimum light detection distance of 5 mm. When a light guide body having a diameter of 35 mm was compared with a light guide body with a diameter of about 10 mmφ for the light detection distance of 35 mm, a larger effect was observed with the light guide body with the diameter of about 10 mmφ. As the light detection distance increases, the propagation distance in the subject increases, and hence the number of times of scattering increases. Therefore, it is generally thought that a detected light intensity decreases with an increase in light detection distance. As shown in FIG. 15, however, when the light guide portion 402 is provided, the intensity of detected light dramatically increases with an increase in light detection distance (in spite of the repeated scattering of light). As the light detection distance increases, the intensity of the light is attenuated. This is a noticeable effect.

### (Combination of Light Guide Functions Using Inclined Structure and Tapered Structure of Detector)

It is also possible to further improve the light detection efficiency while maintaining spatial resolution as much as possible by adjusting both the opening angle φ of each light guide portion 402 and the inclination angle θ of each detector 401 in accordance with the light detection distance R.

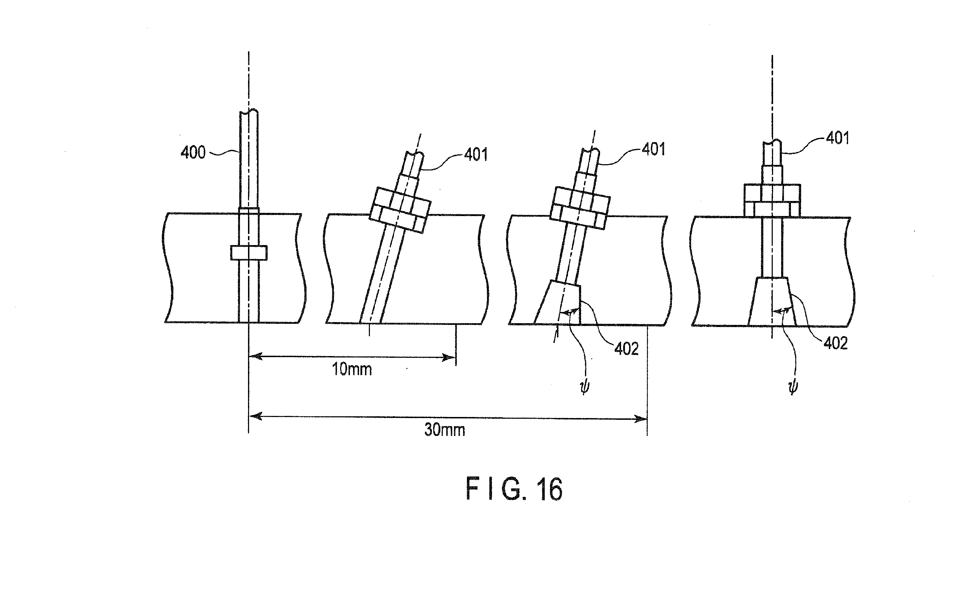

FIG. 16 is a view showing an example of the probe P in which the opening angle φ of each light guide portion 402 and the inclination angle θ of each detector 401 are adjusted in accordance with the light detection distance R. According to the experiments conducted by the present inventors, good optical detection was implemented by adjusting the opening angle φ of each light guide portion 402 and the inclination angle θ of each detector 401 with reference to, for example, light detection distances R = 10 mm and 30 mm, although it depends on the wavelength of light to be used.

That is, in the range of light detection distance R ≤ 10 mm, each detector 401 is inclined so as to decrease the inclination angle θ with an increase in the light detection distance R (that is, so as to incline the detectors 401 more as their distances to the light emission position decrease) while opening angle φ = 0 (or without the light guide portion 402). It was confirmed that in the range of light detection distance R ≤ 10 mm, light emerging from a subject did not become perfect diffusion light but had a certain degree of orientation (angle dependence of emerging light). In this range, therefore, the opening angle of each light guide portion 402 is set to φ = 0 to give priority to the spatial resolution of optical detection over the focusing effect originating from the tapered shape of the light guide portion 402 while improving the detection efficiency by adjusting the inclination angle θ of each detector 401.

In addition, in the range of the light detection distances R given by 10 mm < R ≤ 30 mm, each detector 401 is inclined so as to increase the opening angle φ with an increase in the light detection distance R and decrease the inclination angle θ of each detector 401 with an increase in the light detection distance R. Such an arrangement is used because when the light detection distance R falls within this range, the optical detection efficiency can be expected to be dramatically improved by the tapered shape of the light guide portion 402 and the inclination angle θ of each detector 401.

Furthermore, in the range of light detection distance R > 30 mm, the opening angle φ is increased with an increase in the light detection distance R, and the inclination angle of each detector 401 is set to θ = 0 (that is, the optical axis of the detector 401 is made substantially parallel to the optical axis of the light emission unit 400). Such an arrangement is used in this range, since emerging light is scattered light and has less orientation. The effect of inclination of the detectors 401 with each inclination angle is small. The optical detection efficiency can be expected to be dramatically improved owing to the tapered shape of the light guide portion 402.

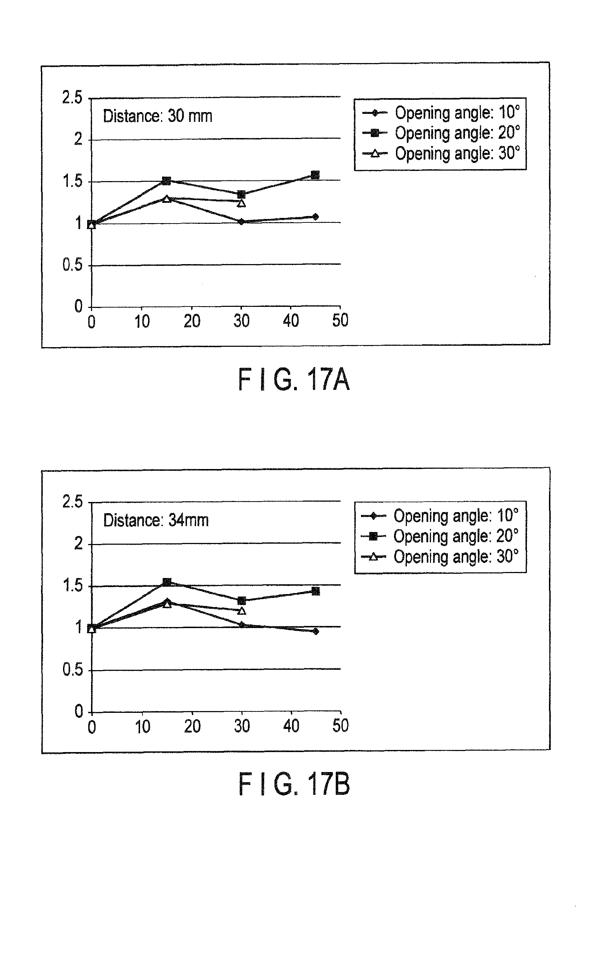

In addition, in order to obtain the optimal opening angle φ of the light guide portion 402 for each light detection distance, the present inventors measured the relationships between the inclination angles θ and the intensities of detected light by using the optical probe 40 shown in FIG. 6 for the respective opening angles of the light guide portion 402, i.e., φ = 10°, 20°, and 30°, when the light detection distances were set to 30 mm and 34 mm. FIGS. 17A and 17B are graphs obtained by this measurement. As is obvious from FIGS. 17A and 17B, detected light exhibited the maximum intensity when opening angle φ = 20° both in cases in which the light detection distances were 30 mm and 34 mm. In addition, the effect of increasing light guide efficiency obtained by the light guide portion 402 described above was observed in the range of the opening angles φ from 10° to 30°.

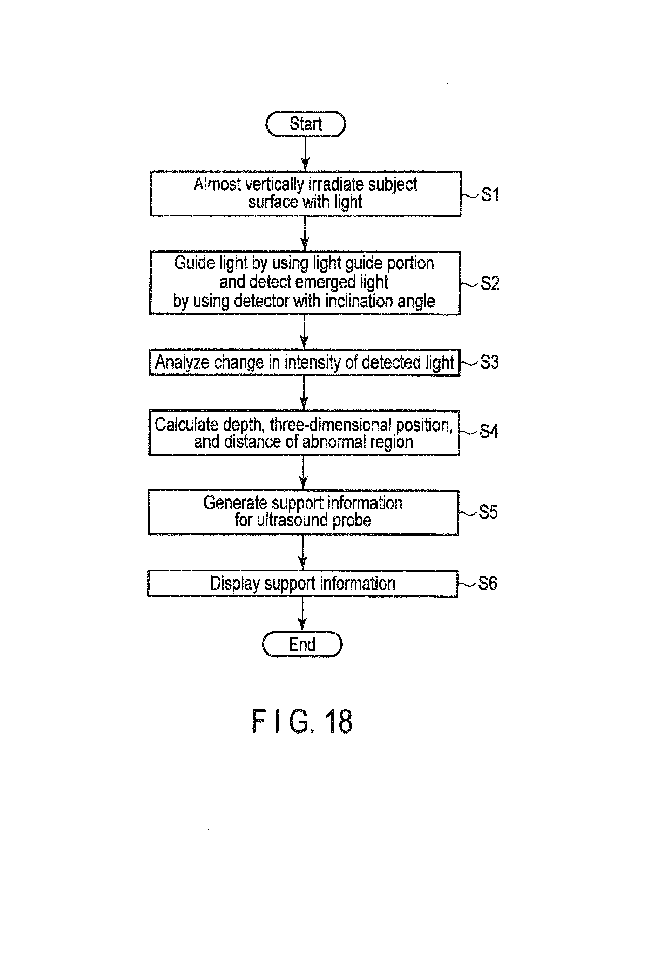

The processing of guiding the ultrasound probe by using the biological optical measurement apparatus 4 according to this embodiment will be described next. FIG. 18 is a flowchart showing the processing of guiding the ultrasound probe by using the biological optical measurement apparatus 4. As shown in FIG. 18, when the light emission unit 400 emits light almost vertically to a subject surface (step S1), the light diffused and reflected within the subject is guided by the plural light guide portions 402 whose opening angles φ differ in accordance with light detection distances, and is detected by the plural detectors 401 each with the individual inclination angle θ (step S2). The optical analyzer 424 amplifies a detected optical signal (analog signal, converts it into a digital signal, and analyzes a change in the intensity of detected light between the detectors 401 (step S3). The computation circuit unit 426 calculates the degree of contact between the ultrasound probe 12 and the subject surface, the depth of an abnormal region exhibiting a predetermined light absorption coefficient in the subject from the subject surface, and the position and distance of the abnormal region with reference to a predetermined position, based on the change in the intensity of detected light between the detectors 401 (step S4).

The support information generating unit 44 generates support information for instructing and guiding the position, posture, direction, and the like of the ultrasound probe 12 based on the determination result and calculation result obtained by the optical measurement processing unit 42 in order to bring the transmitter/receiver surface of the ultrasound probe 12 into contact with the subject (step S5). The monitor 14 displays the generated support information in a predetermined form (step S6).

### (First Modification)

In the above embodiment, the inclination angle θ of each detector 401 and the opening angle φ of each light guide portion 402 are fixed in accordance with a light detection distance. However, without being limited to this example, for example, a moving mechanism capable of arbitrarily adjusting the inclination angle θ and the opening angle φ may be provided, as needed.

### (Second Modification)

The above embodiment has shown the arrangement configured to increase the opening angle φ as a technique of increasing the opening area of the light guide portion 402. However, without being limited to this example, for example, the opening area of the light guide portion 402 may be increased by increasing the height of a circular truncated conical shape forming a tapered shape.

According to the biological optical measurement apparatus described above, the inclination angle θ which is the angle defined by the central axis of the light emission unit and the central axis of each detector is individually set for each detector 401 in accordance with the light detection distance R. In addition, the opening angle φ of the light guide portion having the tapered structure and provided between the detector and the subject surface is set individually for each detector 401 in accordance with the light detection distance R. In specifying light scattering in a subject, since emerging light from the subject is not perfect diffusion light, it is possible to efficiently collect light emerging from the subject upon being scattered repeatedly within the subject, as compared with the related art. Therefore, it is possible to execute biological optical measurement with a higher S/N ratio than the related art, and to determine and show the existence of a suspicious tissue in the subject.

In addition, using the biological optical measurement apparatus according to this embodiment, the ultrasound probe (and the optical probe) can be accurately guided to a desired position (for example, a position just above the suspicious tissue). This reduces variations in the quality of ultrasound image diagnosis caused by variations in the skill level of operators, and can greatly shorten the time required for computation of an optical constant corresponding to metabolic information. In addition, when interpreting an ultrasound image, since it is possible to enforce for the ultrasound image by using the metabolic information obtained by optical measurement, the radiogram interpreter can make quick and proper determination.

Note that, for example, International Publication WO2006/132218 discloses a conventional biological photodetection apparatus. This biological photodetection apparatus has a structure in which a waveguide has different opening areas on the subject side and the detector side to receive light from a subject without omission, and a function of setting a larger opening area on the subject side. This apparatus is designed to detect the light spontaneously emitted from the inside of a subject without emitting light to the subject. In contrast to this, the biological optical measurement apparatus 4 according to this embodiment is based on the new experimental fact that the effect of a light guide body differs depending on the light detection distance (the distance between the emerging position of light entering the subject and the emerging position of light emerging from the subject upon propagating in the subject), and the effective diameter also differs depending on the light detection distance. Therefore, the biological optical measurement apparatus 4 according to the embodiment achieves a remarkable technical effect which cannot be achieved by the conventional biological optical measurement apparatus.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

## Claims

1. A biological optical measurement apparatus **characterized by** comprising:
a light emission unit to be arranged on a surface of a subject and configured to substantially vertically emit light from an emission surface of the light emission unit to the surface of the subject;
plural detectors arranged at different positions around the light emission unit, with at least one of the detectors being inclined at a predetermined inclination angle with respect to a central axis of the light emission unit, and configured to detect respectively intensities of light applied from the light emission unit and propagating in the subject;
an image acquisition unit configured to acquire an image with respect to the subject; and
a calculation unit configured to calculate a relative position between a position of the subject corresponding to the acquired image and a position of the detection of the intensities of light by the plural detectors.

2. The apparatus of claim 1, **characterized in that** at least the one inclined detector is placed within 30 mm from an emission surface of the light emission unit.

3. The apparatus of claim 1 or 2, **characterized in that** the inclination angle falls within a range of 10° to 40°.

4. The apparatus of claim 1, 2 or 3, **characterized in that** the inclination angle decreases with an increase in distance from the light emission unit.

5. The apparatus of claim 1, 2, 3 or 4, **characterized by** further comprising plural light guide bodies each provided for at least one of the plural detectors, interposed between a detection surface and a surface of the subject, and having a contact area on a subject surface side which is larger than an opening area on a detector side.

6. The apparatus of claim 5, **characterized in that** the contact area of each of the plural light guide bodies on the subject surface side increases with an increase in distance from the light emission unit.

7. The apparatus of claim 5, **characterized by** further comprising an optical fiber provided between a detection surface of the detector and the light guide portion,
**characterized in that** an opening area of the light guide portion on a surface side of the subject is larger than an opening area of the optical fiber on the light guide portion side.

8. The apparatus of claim 5, **characterized in that** the plural light guide bodies each have a conical shape with an opening angle more than 10° and less than 30°.

9. The apparatus of claim 5, **characterized in that** the light guide portion is provided for the detection portion, of the plural detectors, which is apart not less than 10 mm from the light emission unit.

10. A biological optical measurement apparatus **characterized by** comprising:
a light emission unit to be arranged on a surface of a subject and configured to substantially vertically emit light form an emission surface of the light emission unit to the surface of the subject;
plural detectors arranged at different positions around the light emission unit and configured to detect respectively intensities of light applied from the light emission unit and propagating in the subject; and
at least one light guide portion provided between at least one of the plural detectors and a surface of the subject and formed such that an opening area on a surface side of the subject becomes larger than an opening area on a detection surface side of the detector.

11. The apparatus of claim 10, **characterized in that** the light guide portion is provided for the detector, of the plural detectors, which is apart not less than 10 mm from the light emission unit.

12. The apparatus of claim 10 or 11, **characterized in that** an opening diameter of the light guide portion on a surface side of the subject increases with an increase in distance from the light emission unit.

13. The apparatus of claim 10, 11 or 12, **characterized by** further comprising an optical fiber provided between a detection surface of the detector and the light guide portion,
**characterized in that** an opening area of the light guide portion on a surface side of the subject is larger than an opening area of the optical fiber on the light guide portion side.

14. The apparatus of claim 10, 11, 12 or 13, **characterized in that** the light guide portion has a tapered structure having a substantially circular truncated conical shape.

15. The apparatus of claim 10, 11, 12, 13 or 14, **characterized in that** the at least one of the light emission unit being inclined at a predetermined inclination angle with respect to a central axis of the light emission unit.

16. The apparatus of claim 15, **characterized in that** the at least one inclined detector is placed within 30 mm from an emission surface of the light emission unit.

17. The apparatus of claim 15, **characterized in that** the inclination angle falls within a range of 10° to 40°.

18. The apparatus of claim 15, **characterized in that** the inclination angle decreases with an increase in distance from the light emission unit.

19. An ultrasound diagnostic apparatus **characterized by** comprising:
an ultrasound probe configured to transmit an ultrasound wave from an ultrasound transmitter/receiver surface to an subject and receive an ultrasound wave reflected inside the subject through the ultrasound transmitter/receiver surface;
an optical probe including a light emission unit to be arranged on a surface of a subject and configured to substantially vertically emit light from an emission surface of the light emission unit to the surface of the object, plural detectors arranged at different positions around the ultrasound transmitter/receiver surface, with a central axis of the ultrasound transmitter/receiver surface in a longitudinal direction being a symmetrical axis, and configured to detect intensities of light reflected within the subject, and plural detectors arranged at different positions around the light emission unit, with at least one of the detectors being inclined at a predetermined inclination angle with respect to a central axis of the light emission unit, and configured to detect respectively intensities of light applied from the light emission unit and propagating in the subject;
an image generating unit configured to generate an ultrasound image by using an ultrasound wave received by the ultrasound probe;
a calculation unit configured to calculate a position and size of an abnormal region exhibiting a predetermined light absorption coefficient in the subject based on the intensity of light detected by each of the detectors; and
a display unit configured to display the ultrasound image indicating the position and size of the abnormal region.

20. An ultrasound diagnostic apparatus **characterized by** comprising:
an ultrasound probe configured to transmit an ultrasound wave from an ultrasound transmitter/receiver surface to an subject and receive an ultrasound wave reflected inside the subject through the ultrasound transmitter/receiver surface;
an optical probe including at least one light emission unit to be arranged on a surface of a subject and configured to substantially vertically emit light from an emission surface of the light emission unit to the surface transmitter/receiver and plural detectors arranged around an ultrasound transmitter/receiver surface and configured to detect intensities of light propagating in the subject and an optical probe including at least one light guide portion provided between at least one of the plural detectors and a surface of the subject and formed such that an opening area on a surface side of the subject becomes larger than an opening area on a detection surface side of the detector;
an image generating unit configured to generate an ultrasound image by using an ultrasound wave received by the ultrasound probe;
a calculation unit configured to calculate a degree of contact between the ultrasound transmitter/receiver surface and the subject surface based on the intensity of light detected by each of the detectors;
a support information generating unit configured to generate support information for supporting operation of the ultrasound probe based on the calculated degree of contact; and
a support information output unit configured to output the support information.
